# EUROPEAN PATENT APPLICATION

(11) **EP 1 652 538 A2**
(43) Date of publication of application: **03.05.2006**
(21) Application number: 05023682.7
(22) Date of filing: 28.10.2005
(51) Int. Cl.: A61L 27/36, A61L 27/54

(54) **Autologous platelet gel on a stent graft**

(30) Priority: 28.10.2004 US 977545
(71) Applicant: Medtronic Vascular, Inc., Santa Rosa, CA 95403 (US)
(72) Inventor: Fernandes, Brian, Roseville, Minnesota 55113 (US); Chu, Jack, Santa Rosa California 95409 (US)
(74) Representative: Zimmermann, Gerd Heinrich

(57) **Abstract**

Methods for ameliorating stent graft migration and endoleak using treatment site-specific cell growth promoting compositions in combination with stent grafts are disclosed. Also disclosed are application of cell growth promoting compositions such as, but not limited to, autologous platelet gel compositions directly to treatment sites before, during or after stent graft implantation. Additional embodiments include medical devices having autologous platelet gel coatings and/or autologous platelet gel delivery devices useful for treating aneurysms.

## Description

### FIELD OF THE INVENTION

Methods for preventing stent graft migration and endoleak using treatment site-specific cell growth promoting factor application devices and related methods are disclosed. Specifically, methods for applying cell growth promoting compositions such as, but not limited to, autologous platelet gel compositions directly to treatment sites before, during or after stent graft implantation are provided. More specifically, medical devices having autologous platelet gel coatings and/or autologous platelet gel delivery devices useful for treating aneurysms are provided.

### BACKGROUND OF THE INVENTION

Aneurysms arise when a thinning, weakening section of an artery wall balloons out to more than 150% of the artery's normal diameter. The most common and deadly of these occur in the aorta, the large blood vessel stretching from the heart to the lower abdomen. A normal aorta is between 1.6 to 2.8 centimeters wide; if an area reaches as wide as 5.5 centimeters, the risk of rupture increases such that surgery is recommended. Aneurysms are asymptomatic and they often burst before the patient reaches the hospital.

Aneurysms are estimated to cause approximately 32,000 deaths each year in the United States. Additionally, aneurysm deaths are suspected of being underreported because sudden unexplained deaths, about 450,000 in the United States alone, are often simply misdiagnosed as heart attacks or strokes while many of them may be due to aneurysms. Aneurysms most often occur in the aorta, the largest artery in the body. Most aortic aneurysms, approximately 15,000/year, involve the abdominal aorta while approximately 2,500 occur in the chest. Cerebral aneurysms occur in the brain and present a more complicated case because they are more difficult to detect and treat, causing approximately 14,000 U.S. deaths per year. Aortic aneurysms are detected by standard ultrasound, computerized tomography (CT) and magnetic resonance imaging (MRI) scans and the increased use of these scanning techniques for other diseases has produced an estimated 200% increase in the diagnosis of intact aortic aneurysms. Approximately 200,000 intact aortic aneurysms are diagnosed each year due to this increased screening alone.

U.S. surgeons treat approximately 50,000 abdominal aortic aneurysms each year, typically replacing the abnormal section with a plastic or fabric graft in an open surgical procedure. A less-invasive procedure that has more recently been used is the stent graft which threads a compressed tubular device to the aneurysm and is expected to span the aneurysm to provide support without replacing a section of the aorta. A vascular graft containing a stent (stent graft) is placed within the artery at the site of the aneurysm and acts as a barrier between the blood and the weakened wall of the artery, thereby decreasing pressure on artery. The less invasive approach of stent grafting aneurysms decreases the morbidity seen with conventional aneurysm repair. Additionally, patients whose multiple medical comorbidities make them excessively high risk for conventional aneurysm repair are candidates for stent grafting. Stent grafts have also emerged as a new treatment for a related condition, acute blunt aortic injury, where trauma causes damage to the artery. There are, however, risks associated with endovascular repair of abdominal aortic aneurysms. The most common of these risks is migration of the stent graft due to hemodynamic forces within the artery. Graft migrations lead to endoleaks, a leaking of blood into the aneurysm sac between the outer surface of the graft and the inner lumen of the blood vessel.

The abdominal aorta between the renal artery and the iliac branch is the most susceptible arterial site to aneurysms. While this area of the aorta is ideally straight, in many patients the aorta is curved leading to asymmetrical hemodynamic forces. When a stent graft is deployed in this curved portion of the aorta, hemodynamics place uneven forces on the graft, leading to graft migration. Additionally, the asymmetrical hemodynamic forces can cause remodeling of the aneurysm sac which leads to increased risk of aneurysm rupture and increased endoleaks.

The goal of endovascular repair of aorta aneurysms is to provide a graft which comes in close contact with the vessel wall, in fact sealed to the vessel wall. The greater the area of the stent graft in contact with the artery wall, the better graft fixation, and the tighter seal leads to a decreased risk of migration and endoleak. Endoleaks present a risk factor for post-surgical rupture of the aneurysm due to increased blood pressure within the aneurysm sac.

Stent grafts have been designed with stainless steel anchoring barbs that engage the aortic wall directly to prevent migration. Additionally, endostaples have been developed to fix the graft more readily to the treatment site. These physical anchoring have proven to be effective in some patients however they have not sufficiently ameliorated the graft migration and endoleak problems associated with current stent-grafting methods and devices in all cases.

The combination of the metal scaffolding of most stent grafts and a predilection to graft migration has led to the contraindication of magnetic resonance imaging (MRI) in some patients having stent grafts. The magnetic fields used in this imaging process, when moving across the body, may cause insufficiently endothelialized metal-containing stents to migrate. Anchoring the stent graft into the vessel wall may be expected to ameliorate this problem to the extent that sufficient tissue in-growth occurs. Inducing significant endothelialization of the stent graft may allow patients access to this vital medical diagnostic procedure.

Therefore there exists a medical need for compositions useful for coating stent grafts or direct application to the aneurysm wall at the time of stent implantation that promote healing, reduce endoleaks and minimize device migration by promoting endothelial tissue in-growth. It is an object of the present invention to provide such compositions.

### SUMMARY OF THE INVENTION

This object is solved by a stent graft according to claim 1, and a delivery catheter according to claim 15. Further features, details and advantages of the present invention are apparent from the dependent claims, the specification and the accompanying drawings. Particularly, compositions are provided in combination with vascular stent grafts for the treatment of aneurysms. Additionally, devices are described which provide structural support for weakened arterial walls while the accompanying compositions seal the support to the tissue wall and promote tissue in-growth to reduce graft migration and prevent endoleaks.

In one embodiment according to the present invention, a stent graft is provided comprising a luminal wall contacting surface; a blood flow contacting surface; and a cell growth promoting composition on the luminal wall contacting surface. The stent graft can optionally have a metal scaffold attached to the luminal wall contacting surface of the stent graft. The cell growth promoting compositions may be applied directly to the exterior of the stent graft either alone or dispersed in a biocompatible polymer by spraying, dipping or rolling.

In another embodiment according to the present invention, the cell growth promoting composition is autologous platelet gel (APG).

In yet another embodiment according to the present invention, the stent graft is coated with APG prior to deployment by depositing platelet poor plasma and thrombin on the stent graft in the stent graft chamber of a stent deployment catheter.

In an embodiment according to the present invention, the cell growth promoting composition is an endothelial cell promoting factor, a smooth muscle cell promoting factor or a fibroblast promoting factor such as a vascular growth factor or a heparin binding growth factor.

In another embodiment according to the present invention, the cell growth promoting factors is selected from the group including vascular endothelial growth factor A, vascular endothelial growth factor B, vascular endothelial growth factor C, vascular endothelial growth factor D, placental growth factor, fibroblast growth factor 1, fibroblast growth factor 2 and insulin-like growth factor.

In an embodiment according to the present invention, a method is claimed for providing a stent graft and a cell growth promoting composition comprises administering a cell growth promoting composition directly to a lumen of a blood vessel wall adjacent to a treatment site; and contacting the cell growth promoting composition with the luminal wall contacting surface of the stent graft and the blood vessel luminal wall at the treatment site. The cell growth promoting composition may be autologous platelet gel.

In another embodiment according to the present invention, a further step is included wherein a drug is delivered in combination with the autologous platelet gel. The drug can be selected from the group consisting of small molecules, peptides, proteins, hormones, DNA or RNA fragments, cells, genetically engineered cells, genes, cell growth promoting compositions and matrix metalloproteinase inhibitors.

In yet another embodiment according to the present invention includes a method for providing a stent graft and a cell growth promoting composition comprising advancing a stent deploying catheter containing a vascular stent to a treatment site; advancing at least one injection catheter containing the cell growth promoting composition to the treatment site; deploying the stent graft at the treatment site; and applying the cell growth promoting composition from the injection catheter to the inner lumen of the blood vessel at the treatment site; such that the luminal wall contacting surface of the stent graft engages the cell growth promoting composition at the treatment site. The stent deployment catheter and the injection catheter can be deployed to the treatment site via the same or a different route. Additionally, the injection catheter can reach the treatment site via a blood vessel which bisects the treatment site. The injection catheter can be a single lumen injection catheter or a multilumen injection catheter.

In an embodiment according to the present invention, the treatment site is the area where the distal end of the stent graft contacts the vessel lumen wall. In another embodiment according to the present invention, the treatment site is the junction between a stent graft and an iliac limb section. In yet another embodiment according to the present invention, the treatment site is the aneurysm sac.

In one embodiment according to the present invention, a delivery catheter is provided for delivering a stent and a cell growth promoting composition to an aneurysm site which comprises a stent graft chamber in which the stent graft is radially compressed; an injection catheter disposed within the delivery catheter; and at least one injection port at the distal end of the injection catheter capable of delivering a cell growth promoting composition. The injection catheter can be a single lumen injection catheter or a multilumen injection catheter. The injection catheter may further comprise a vent for expressing air or excess cell growth promoting composition.

In an embodiment according to the present invention, a method is provided for treating abdominal aortic aneurysms comprising delivering a stent graft to a treatment site; promoting endothelialization of the stent graft by depositing a cell growth promoting composition on the inner lumen of the blood vessel contacting the luminal wall contacting surface of the stent graft and the inner lumen of the blood vessel; and promoting strengthening and fixation of stent graft by enhancing proliferation of smooth muscle cells and fibroblasts. The cell growth promoting composition for treating abdominal aortic aneurysms may be autologous platelet gel.

### BRIEF DESCRIPTION OF THE FIGURES

FIG. 1 depicts a fully deployed stent graft with an exterior metal scaffolding as used in one embodiment according to the present invention.

FIG. 2a-b depict a stent graft delivery catheter containing a multilumen injection catheter for coating the stent graft with autologous platelet gel immediately prior to deployment in accordance with the teachings according to the present invention.

FIG 3a-c depicts deployment of a stent graft and an multilumen injection catheter suitable for injection of a cell growth promoting factor simultaneously in accordance with the teachings according to the present invention.

FIG 4a-d depicts a method of injection of APG directly into the aneurysm sac after deployment of a stent graft in accordance with the teachings according to the present invention.

FIG. 5a-c depicts an alternate method of injection of APG directly into the aneurysm sac after deployment of a stent graft in accordance with the teachings according to the present invention.

FIG. 6 depicts an alternate method of injection of APG directly into the aneurysm sac after deployment of a stent graft in accordance with the teachings according to the present invention.

FIG. 7 depicts the effects of the autologous platelet gel according to the present invention on arterial smooth muscle cell proliferation.

FIG. 8 depicts the effects of the autologous platelet gel according to the present invention on endothelial cell proliferation.

FIG. 9 depicts the effects of the autologous platelet gel according to the present invention on fibroblast cell proliferation.

FIG. 10 depicts the effects of platelet poor plasma on human dermal fibroblast growth.

FIG. 11 depicts the effects of the autologous platelet gel according to the present invention on endothelial cell migration.

FIG. 12 depicts the tissue response to implantation of the autologous platelet gel according to the present invention or Matrigel® in athymic mice.

### DEFINITION OF TERMS

Prior to setting forth embodiments according to the present invention, it may be helpful to an understanding thereof to set forth definitions of certain terms that will be used hereinafter:

Animal: As used herein "animal" shall include mammals, fish, reptiles and birds. Mammals include, but are not limited to, primates, including humans, dogs, cats, goats, sheep, rabbits, pigs, horses and cows.

Biocompatible: As used herein "biocompatible" shall mean any material that does not cause injury or death to the animal or induce an adverse reaction in an animal when placed in intimate contact with the animal's tissues. Adverse reactions include inflammation, infection, fibrotic tissue formation, cell death, or thrombosis.

Cell Growth Promoting Compositions: As used herein "cell growth promoting factors" or "cell growth promoting compositions" shall include any bioactive compound having a growth promoting effect on vascular cells. Exemplary, non limiting examples include, vascular endothelial growth factor (VEGF), platelet-derived growth factor (PDGF), plated-derived epidermal growth factor (PDEGF), fibroblast growth factors (FGFs), transforming growth factor-beta (TGF-β), platelet-derived angiogenesis growth factor (PDAF) and autologous platelet gel (APG).

Drug(s): As used herein "drug" shall include any bioactive compound or composition having a therapeutic effect in an animal. Exemplary, non limiting examples include small molecules, peptides, proteins, hormones, DNA or RNA fragments, genes, cells, genetically-modified cells, cell growth promoting compositions, matrix metalloproteinase inhibitors and autologous platelet gel.

Endoleak: As used herein "endoleak" refers to Type I endoleak, i.e., the presence of flow of blood past the seal between the proximal end of the stent graft and the vessel wall, and into the aneurysmal sac, when all such flow should be contained within its lumen.

Heparin Binding Growth Factor Family: As used herein "heparin binding growth factor family shall include factors binding heparin and having a positive growth effect on vascular cells. Exemplary, non limiting examples include fibroblast growth factor 1 (FGF-1), FGF-2 and insulin-like growth factor.

Migration: As used herein "migration" refers to displacement of the stent graft sufficient to be associated with another complication, for example, endoleak.

Treatment Site: As used herein "treatment site" shall mean an aneurysm site, acute traumatic aortic injury or other vascular-associated pathology.

Vascular Growth Factor: As used herein "vascular growth factor" shall include factors having a positive effect on growth of vascular cells. Exemplary, non limiting examples include vascular endothelial growth factor A (VEGF-A), VEGF-B, VEGF-C, VEGF-D and placental growth factor.

### DETAILED DESCRIPTION

Embodiments according to the present invention provide compositions, devices and related methods useful for preventing implantable medical device post-implantation migration and endoleak. More specifically, compositions, devices and related methods promote implantable medical device attachment to blood vessel luminal walls. One embodiment provides methods and compositions useful for minimizing post-implantation stent graft migration following deployment at an aneurysmal treatment site and is also useful in preventing or minimizing post-implantation endoleak following stent-graft deployment at an aneurysmal treatment site.

For convenience, the devices, compositions and related methods according to the present invention discussed hereinafter will be exemplified using stent grafts intended to treat aneurysms. As discussed briefly above, an aneurysm is swelling, or expansion of the blood vessel lumen at a defined point and is generally associated with a vessel wall defect. Aneurysms are often a multi-factorial asymptomatic vessel disease that if left unchecked may result in spontaneous rupture, often with fatal consequences. Previous methods to treat aneurysms involved highly invasive surgical procedures where the affected vessel region is removed and replaced with a synthetic graft that is sutured in place. However, this procedure is extremely risky and is generally only employed in otherwise healthy vigorous patients who are expected to survive the associated surgical trauma. Elderly and more feeble patients were not candidates for many aneurysmal surgeries and remained untreated and thus at continued risk for sudden death.

In order to overcome the risks associated with invasive aneurysmal surgeries, stent grafts were developed that can be deployed with a cut down procedure or percutaneously using minimally invasive procedures. Essentially, a catheter having a stent graft compressed and fitted into the catheter's distal tip is advanced through an artery to the aneurysmal site. The stent graft is then deployed within the vessel lumen juxtaposed to the weakened vessel wall forming an inner liner that insulates the aneurysm from the body's hemodynamic forces thereby reducing, or eliminating the possibility of rupture. The size and shape of the stent graft is matched to the treatment site's lumen diameter and aneurysm length. Moreover, branched grafts are commonly used to treat abdominal aortic aneurysms that are located near the iliac branch.

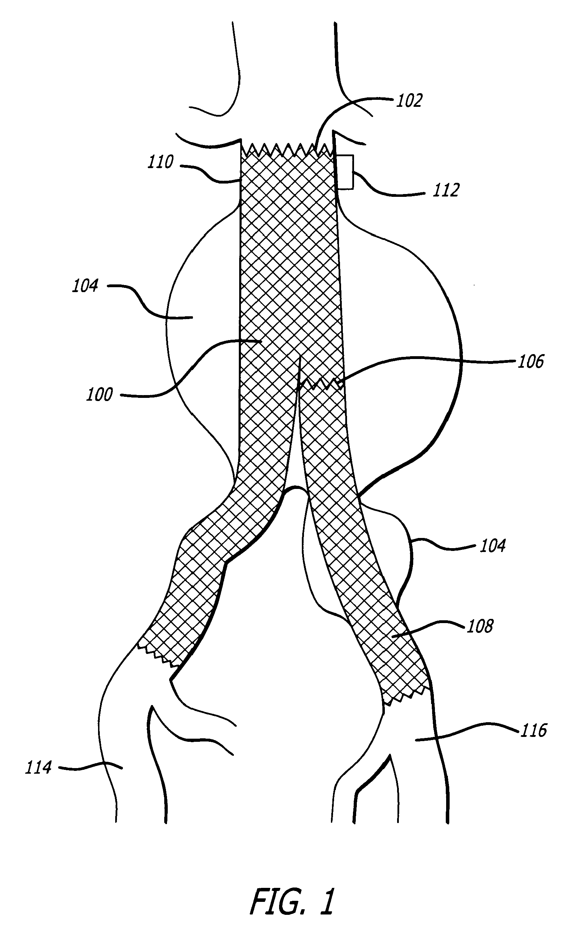

Stent grafts generally comprise a metal scaffolding (see **114** in FIG. 1) having a biocompatible covering such a Dacron® or a fabric-like material woven from a variety of biocompatible polymer fibers. Other embodiments include extruded sheaths and coverings. The scaffolding is generally on the luminal wall-contacting surface of the stent graft and directly contacts the vessel lumen. The sheath material is stitched, glued or molded onto the scaffold. In other embodiments, the scaffolding may be on the graft's blood flow contacting surface or interior. When a self-expanding stent graft is deployed from the delivery catheter, the scaffolding expands to fill the lumen and exerts circumferential force against the lumen wall. This circumferential force is generally sufficient to keep the stent-graft from migrating and to minimize endoleak. However, stent migration and endoleak may occur in vessels that have irregular shapes or are shaped such that they exacerbate hemodynamic forces within the lumen. Stent migration refers to a stent graft moving from the original deployment site, usually in the direction of the blood flow. Endoleak (Type I) refers to the seepage of blood around the stent ends to pressurize the aneurismal sac or between the stent graft and the lumen wall. Stent graft migration may result in the aneurysmal sac being exposed to blood pressure again and increasing the risk of rupture. Endoleaks (of all types) occur in 10-25% of aneurysms treated with stent grafts. Some surgeons believe that endoleaks increase the risk of aneurysm expansion or rupture. Therefore, it would be desirable to have devices, compositions and methods that minimize post implantation stent graft migration and endoleak.

In one embodiment according to the present invention, cell growth promoting compositions are administered to a treatment site within a vessel lumen, either before, during or after stent graft implantation. The vessel wall's blood-contacting lumen surface comprises a layer of endothelial cells. In the normal mature vessel the endothelial cells are quiescent and do not multiply. Thus, a stent graft carefully placed against the vessel wall's blood-contacting luminal surface rests against a quiescent endothelial cell layer. However, if cell growth promoting compositions are administered immediately before, during or immediately after stent graft deployment, the normally quiescent endothelial cells lining the vessel wall, and in intimate contact with the stent graft luminal wall contacting surface, will be stimulated to proliferate. The same will occur with smooth muscle cells and fibroblasts found within the vessel wall. As these cells proliferate they will grow into and around the stent graft lining such that the stent graft becomes physically attached to the vessel lumen rather than merely resting against it. In one embodiment according to the present invention, the stent graft has a metallic scaffolding on the graft's luminal wall contacting surface and the cell growth promoting factor is autologous platelet gel (APG).

Autologous platelet gel is formed from autologous platelet rich plasma (PRP) mixed with thrombin and calcium. The PRP is generated from variable speed centrifugation of autologous blood using devices such as, but not limited to, Medtronic Inc.'s Magellan™ Autologous Platelet Separation System. The PRP contains sufficient fibrinogen to allow a fibrin gel to form when mixed with calcium and thrombin. In addition, the PRP contains a high concentration of platelets that can aggregate for plugging, as well as release cytokines, growth factors or enzymes following activation by thrombin. Some of the many factors released by the platelets and the white blood cells present that constitute the PRP include platelet-derived growth factor (PDGF), plated-derived epidermal growth factor (PDEGF), fibroblast growth factor (FGF), transforming growth factor-beta (TGF-β) and platelet-derived angiogenesis growth factor (PDAF). These factors have been implicated in wound healing by increasing the rate of collagen secretion, vascular in-growth and fibroblast proliferation.

Implantable medical devices, specifically stent grafts, are advantageously sealed to the vessel lumen using APG. The APG comprises platelet aggregates which help mechanically seal the stent graft to the lumen wall in addition to providing a rich source of growth factors. Briefly, following activation by thrombin, platelets release thromboxane A2, adenosine diphosphate and thrombin, factors that attract additional platelets to the developing clot. Once associated with the stent graft, APG, with its rich composition of growth and healing factors, can promote the integration of the stent graft into the vessel wall. Enhanced healing and tissue in-growth from the surrounding vessel may lessen the chances for graft migration and endoleak. Additionally, drugs that inhibit matrix metalloproteinases, or other pathological processes involved in aneurysm progression, can be incorporated into the gel to enhance wound healing and/or stabilize and possibly reverse the pathology. Drugs that induce positive effects at the aneurysm site can also be delivered by APG and the methods described.

Autologous platelet gel is injectable and can be generated and applied to the stent graft in the operating room immediately prior to deployment. The stent graft can be coated with APG by dipping the stent in a receptacle containing the forming gel; using a modified version of a standard delivery catheter to deliver the APG or injecting the APG directly into the aneurysm site to prevent pressure build-up. Single lumen catheters may be used to deliver either the components of APG or pre-formed APG. or multilumen catheters may be used to deliver the PRP and calcium/thrombin activating solution concurrently to the aneurysm site.

Because of the physical properties of APG, it may be particularly useful in promoting endothelialization of vascular stent grafts. The APG not only can coat the exterior surface of the stent graft but also fills the pores, inducing migrating cells into the stent graft fabric. As a result, engraftment of autologous endothelial cells will occur preferentially at those sites where APG was injected. Previously, vascular prostheses were seeded with non-autologous materials, enhancing the possibility of graft rejection. Autologous platelet gel will not cause antigenicity or rejection effects. Additionally the APG may fill gaps between the stent graft outer wall and the inner lumen of the aneurysm sac further preventing endoleaks and providing structural support for weakened arterial walls within the aneurysm sac.

Endothelialization has been observed to naturally occur in few human coronary stents within weeks of implantation. This natural endothelialization is not complete or consistent, however, and does not prevent the stent graft side effects of graft migration and endoleak. Methods to increase endothelialization are sought to improve clinical outcome after stent grafting.

Endothelialization may be stimulated by induced angiogenesis resulting in formation of new capillaries in the interstitial space and surface endothelialization. This has led to modification of medical devices with vascular endothelial growth factor (VEGF) and fibroblast growth factors 1 and 2 (FGF-1, FGF-2). The discussion of these factors is for exemplary purposes only, as those of skill in the art will recognize that numerous other growth factors have the potential to induce cell-specific endothelialization. VEGF is endothelial cell-specific however it is a relatively weak endothelial cell mitogen. FGF-1 and FGF-2 are more potent mitogens but are less cell specific. The development of genetically-engineered growth factors is anticipated to yield more potent endothelial cell-specific growth factors. Additionally it may be possible to identify small molecule drugs that can induce endothelialization.

Therefore embodiments according to the present invention provide coatings for stent grafts that incorporate endothelialization factors other than APG. Drug-eluting stents have been developed for treatment of restenosis, which results from cardiac stent implantation. Stent grafts can be coated with endothelialization factors, including growth factors and drugs. The field of medical device coatings is well established and methods for coating stent grafts with bioactive compositions, with or without added polymers, is well known to those of skill in the art.

In some embodiments, the stent graft is provided "pre-loaded" into a deployment catheter and thus cannot be pre-coated with APG. Consequently, APG must be applied to the stent graft, luminal wall or both, contemporaneous with stent graft deployment. In normal stent deployment protocols, a vascular stent graft **100** is fully deployed through the left iliac artery **114** to an aneurysm site **104** (FIG. 1). Stent graft **100** has a distal end **102** and an iliac leg **108** to anchor the stent graft in the iliac artery. Stent graft **100** is deployed first in a first deployment catheter and iliac leg **108** is deployed in a second deployment catheter. The stent graft **100** and iliac leg **108** are joined with a 2 cm overlap of the two segments

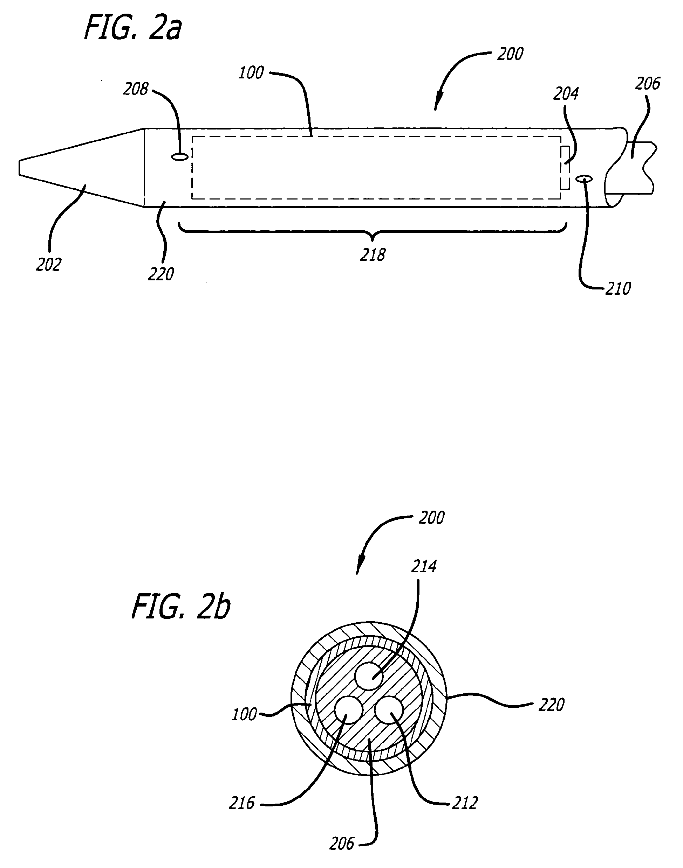

In an embodiment according to the present invention, a stent graft is pre-loaded into a delivery catheter such as that depicted in FIG. 2a. Stent graft **100** is radially compressed to fill the stent graft chamber **218** in the distal end of catheter **200.** The stent graft **100** is covered with a retractable sheath **220.** Within the lumen of catheter **200** is a multilumen injection catheter **206.** Injection catheter **206** (FIG. 2b) has a guide wire lumen **212,** a lumen **214** for delivery of PRP and a lumen **216** for delivery of thrombin. Catheter **206** has two injection ports **208** and **210** for delivering the PRP and thrombin (or other cell growth promoting factors) contemporaneously with stent graft deployment. In this embodiment, PRP is injected through injection port **208** to wet stent **100.** Thrombin is next injected through injection port **210** to react with PRP to form APG on the surface of stent graft **100** within stent graft chamber **218.** Stent graft **100** is then deployed to the treatment site as depicted in FIG. 1.

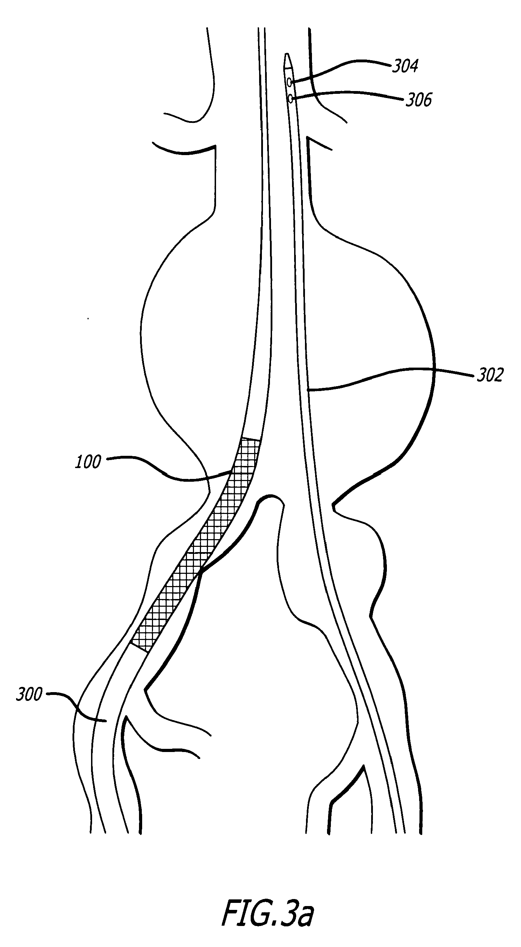

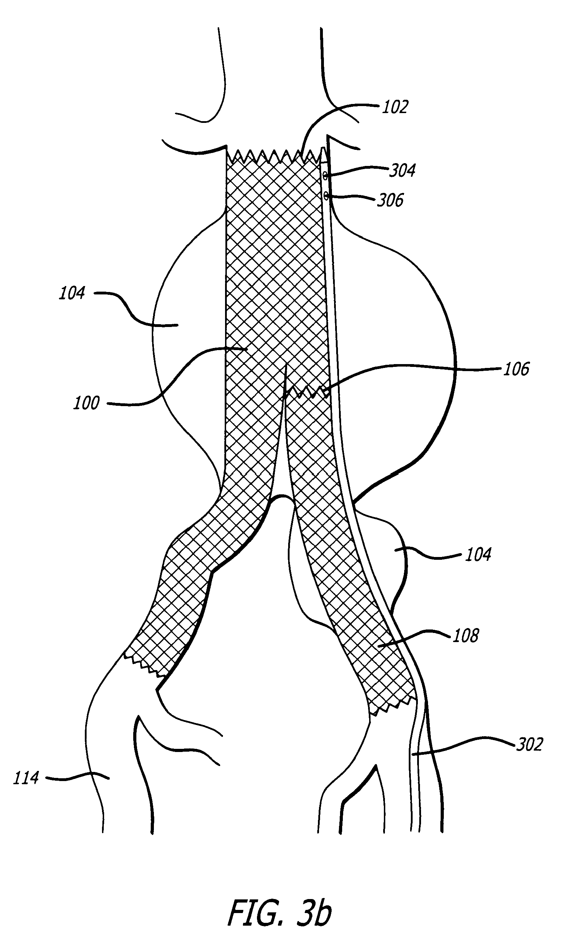

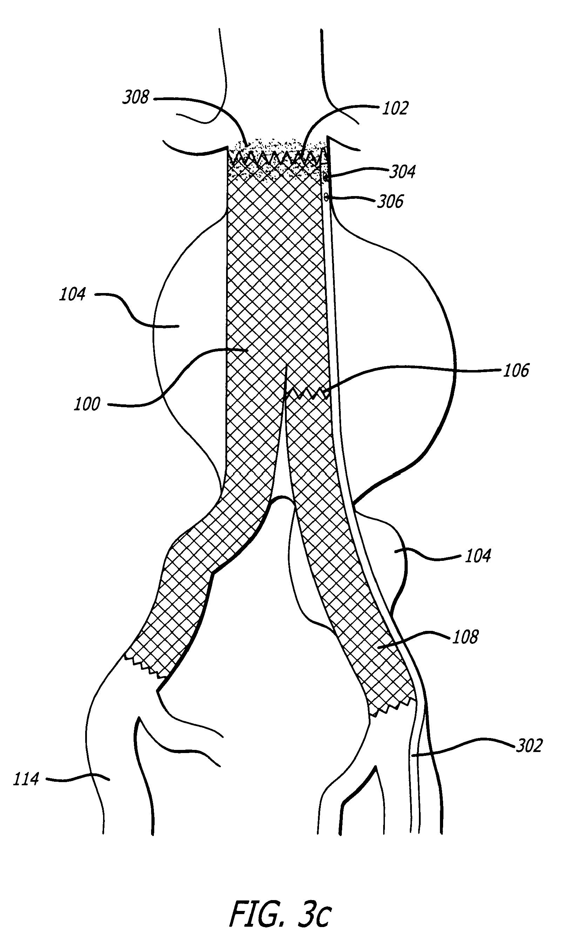

In another embodiment, APG is injected between the stent graft and the vessel wall during stent graft placement. As depicted in FIG. 3a, a stent graft **100** is radially compressed to fill the stent graft chamber **218** of stent delivery catheter **300** which is then deployed to the treatment site via the left iliac artery **114.** A multilumen injection catheter **302** is also deployed to the treatment site through the right iliac artery **116.** The multilumen injection catheter **302** can be a coaxial catheter with two injection lumens or a dual lumen catheter or alternatively a three lumen catheter if a guide wire lumen is required. Injection catheter **302** has injection ports **304** and **306** through which PRP and thrombin may be deposited. In the first step of this deployment scheme (FIG. 3a), the stent delivery catheter **300** and the injection catheter **302** are deployed independently to the treatment site and the stent **100** is deployed. Delivery catheter **300** is removed and the iliac limb **108** is deployed as in FIG. 1 while the injection catheter **302** remains in place (FIG. 3b) with its injection ports **304** and **306** aligned with the distal end **102** of stent graft **100.** Thrombin and PRP are injected simultaneously between the vessel lumen wall and the stent graft at the distal end **102** of stent graft **100** to form APG **308** (FIG. 3c). The injection catheter **302** is then retrieved. This same procedure can be repeated as necessary to apply APG to the stent graft and/or luminal wall at other locations as depicted in FIG. 1 (102, 112 and 106).

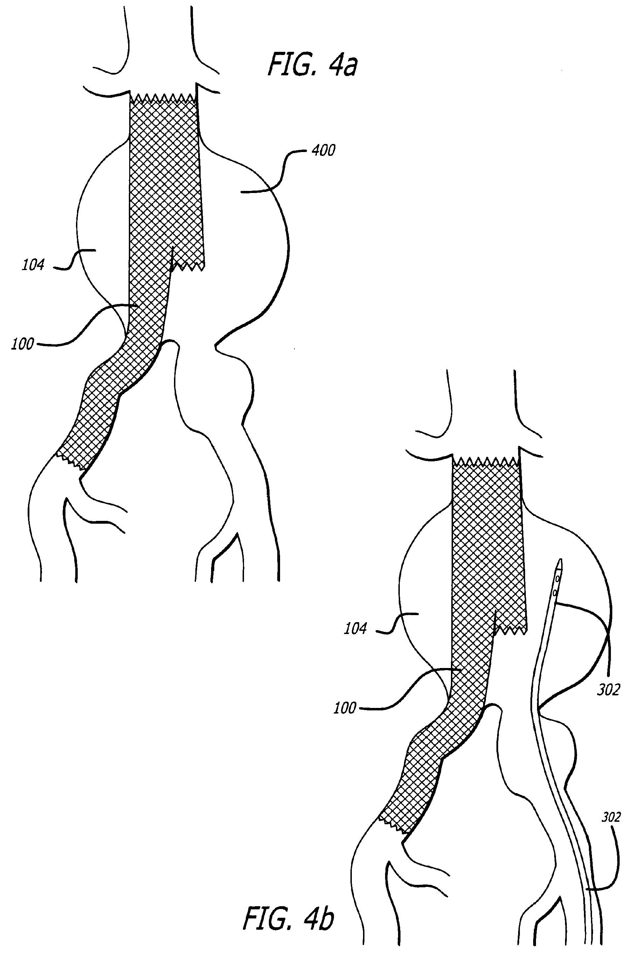

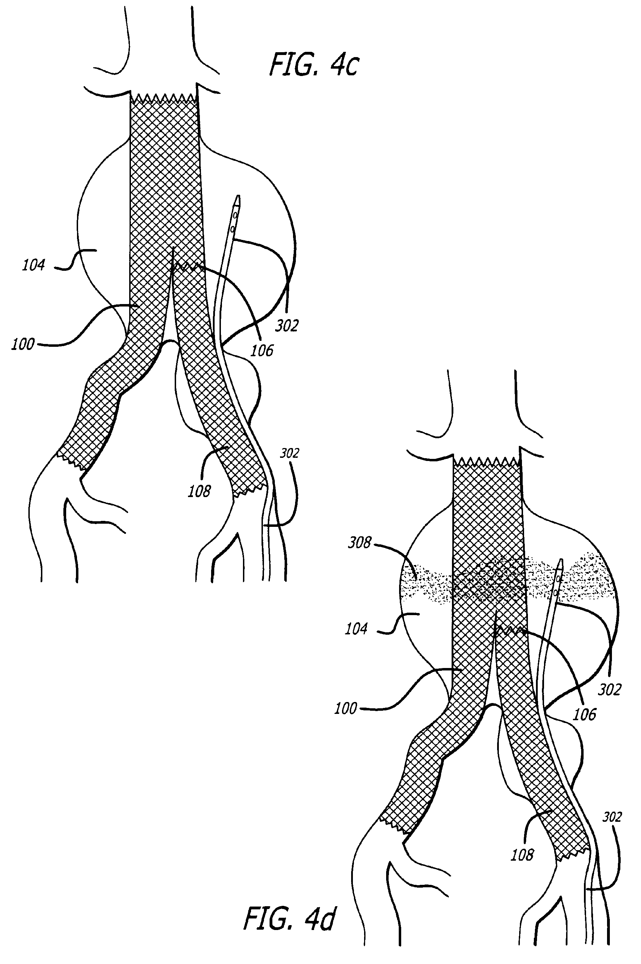

In another embodiment, APG is delivered directly to the aneurysm sac. As previously described, stent graft 100 is radially compressed to fill the stent graft chamber 218 of stent delivery catheter 300 which is then deployed to the treatment site via the left iliac artery 114 (FIG. 4a). An injection catheter 302 is also deployed to the aneurysm sac through the right iliac artery 116 (FIG. 4b). The injection catheter 302 can be a coaxial catheter with two injection lumens or a dual lumen catheter or alternatively a three lumen catheter if a guide wire lumen is required. Injection catheter 302 has injection ports 304 and 306 through which PRP and thrombin may be deposited. Delivery catheter 300 is removed and the iliac limb 108 is deployed as in FIG. 1 while the injection catheter 302 remains in place (FIG. 4c) with its injection ports 304 and 306 in the aneurysm sac 400. The iliac limb segment of the stent graft seals the aneurysm sac at the proximal end. Thrombin and PRP are injected simultaneously between the vessel lumen wall and the stent graft within the aneurysm sac to form APG 308 (FIG. 4d). An amount of PRP and thrombin necessary to produce enough APG to fill the aneurysm sac is then deployed. The injection catheter 302 is then retrieved.

In another embodiment, single lumen injection catheters can be used in the place of multilumen injection catheter 302. After the guide wire is retrieved from the lumen, PRP and thrombin can be delivered to the treatment site sequentially through the same lumen of the single lumen injection catheter. In an alternate embodiment, more than one single lumen injection catheters can be deployed in each iliac artery with the distal ends of the catheters meeting in the aneurysm sac. Thrombin and PRP can then each be injected through the single lumen injection catheters to form APG in the aneurysm sac.

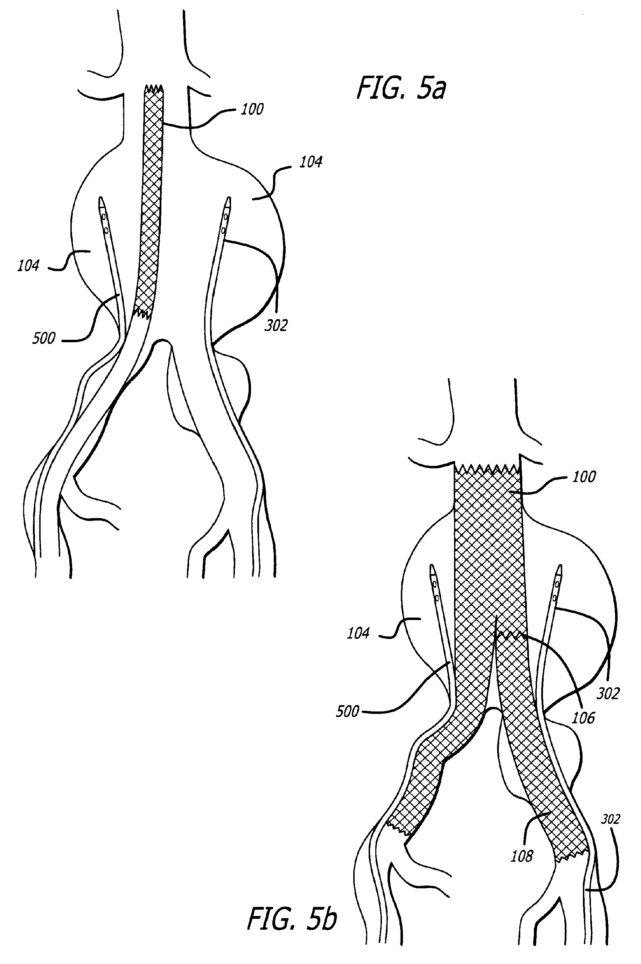

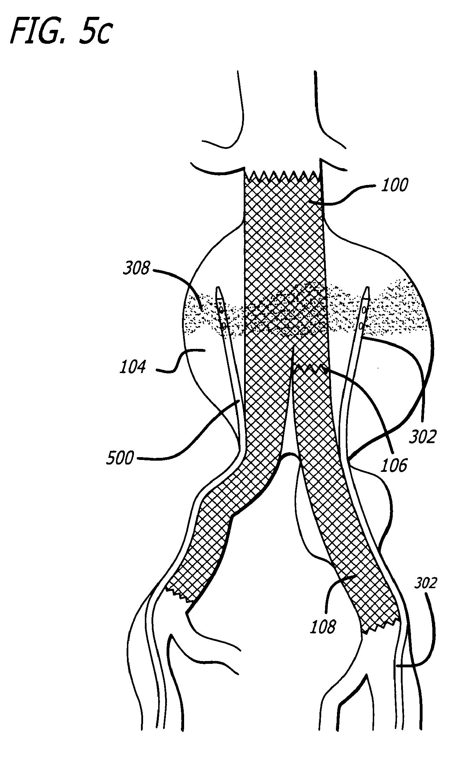

In an alternative embodiment, more than one injection catheter can be used to form APG within the aneurysm sac (FIG.5). As previously described, stent graft 100 is radially compressed to fill the stent graft chamber 218 of stent delivery catheter 300 which is then deployed to the treatment site via the left iliac artery 114 (FIG. 5a). Multiple single lumen or multilumen injection catheters 302 and 500 are also deployed to the aneurysm sac through the right iliac artery 116 and left iliac artery 118 (FIG. 4a). Injection catheters **302** and **500** have injection ports through which PRP and thrombin may be deposited. Delivery catheter **300** is removed and the iliac limb **108** is deployed as in FIG. 1 while injection catheters **302** and **500** remain in place (FIG. 5b) with their injection ports **304** and **306** in the aneurysm sac **400.** The iliac limb segment of the stent graft seals the aneurysm sac at the proximal end. Thrombin and PRP are injected simultaneously between the vessel lumen wall and the stent graft within the aneurysm sac to form APG **308** (FIG. 5c). An amount of PRP and thrombin necessary to produce enough APG to fill the aneurysm sac and seal the ends is determined radiographically by measuring the size of the aneurysm sac prior to surgery. The injection catheters **302** and **500** are then retrieved.

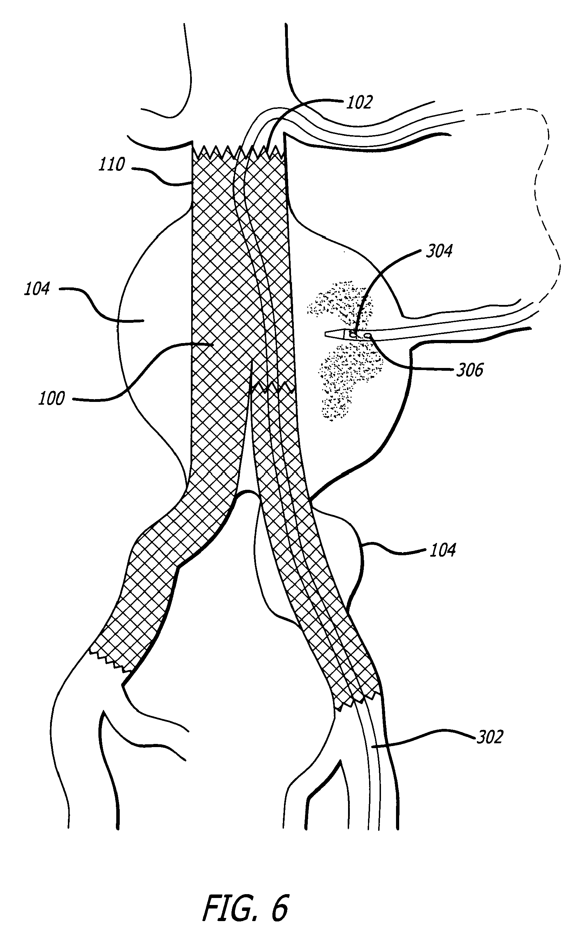

In yet another embodiment, depending on stent placement, a collateral artery can be used to access the luminal wall-contacting surface of a deployed stent graft (FIG. 6). For example, and not intended as a limitation, stent graft **100** may be deployed such that the distal end **102** is in the abdominal aorta near, but below the renal artery. After deployment of stent graft **100,** deployment catheter **200** is removed and an injection catheter **302** is advanced up the aorta past the treatment site **104** to the superior mesenteric artery. The injection catheter **302** is then advanced through the superior mesenteric artery and down into the inferior mesenteric artery where it originates at the aorta immediately distal to the treatment site **104** and the distal end of the stent graft **102.** The APG is then injected at a site adjacent the lumen wall/stent graft interface (see FIG. 1 at **112)** or into the aneurysm sac **400** and allowed to diffuse into and around the stent graft **100.**

Once the APG, or alternate cell growth promoting factor, or combinations thereof, has been administered to the stent graft/vessel lumen interface, cell growth will be activated and cells will proliferate and adhere to the stent graft (a condition or process referred to herein after as "tissue in-growth" or endothelialization) thus anchoring the stent graft securely to the vessel lumen and preventing stent graft migration. Moreover, tissue in-growth will also provide a seal between the distal end of the luminal wall contacting surface of stent graft **102** or other locations at risk for endoleak (**102**, **112** and **106).**

In another embodiment, the cell growth promoting compositions are stably pre-coated on stent grafts. Drug-eluting vascular stents are well known in the field for preventing and treating restenosis secondary to cardiac stent implantation. Delivery of solubilized drugs to sites within the vasculature has been attempted using weeping balloon and injection catheters without success. The blood flow quickly flushes the drug down stream and away from the treatment site. For this reason, methods of delivery of drugs coated on stents or administered in a gel were developed. Drug-coated stent grafts are manufactured by spraying, rolling or dipping the stent in a solution containing the drug. Alternatively, depending on the characteristics of the drug, it may be desirable to coat the stent graft with the composition dispersed in a suitable polymer coating. Biocompatible and hemocompatible polymers, including, but not limited to, poly(ethylene glycol) (PEG)-containing polymers, are known to those of skill in the polymer coating art. Cell growth promoting compositions including, but not limited to, VEGF, FGF-1 and FGF-2, can be stably coated onto stent grafts by spraying, rolling or dipping the stent graft in solutions containing the cell growth promoting factor with or without coating polymers.

The following examples are meant to illustrate one or more embodiments according to the invention and are not meant to limit the scope of the invention to that which is described below.

### Example 1

### Properties of Platelet Rich Plasma

Aliquots of human peripheral blood (30-60 mL) are passed through the Magellan™ Autologous Platelet Separation System (the Magellan™ system) and the concentrated, platelet-rich plasma fraction (PRP) assayed for platelets (PLT), white blood cells (WBC) and hematocrit (Hct) (Table 1). The Magellan™ system concentrated platelets and white blood cells six-fold and three-fold respectively.

**Table 1. Blood cell yields after passing through the Magellan™ system.**

| **Mean ± SD n=19** | **Initial Blood** | **PRP** | **Yield** |
|---|---|---|---|
| PLT(x1000/µL) | 220.03 ± 48.58 | 1344.89 ± 302.00 | 6.14 ± 0.73 |
| WBC(x1000p/L) | 5.43 ± 1.43 | 17.04 ± 7.01 | 3.12 ± 0.90 |
| Hct (%) | 38.47 ± 2.95 | 6.81 ± 1.59 | |

| | | | |
|---|---|---|---|
| Cell Yield = cell count in PRP/cell count in initial blood = [times baseline] | | | |

Additionally, PRP was assayed for levels of the endogenous growth factors platelet-derived growth factor (PDGF), transforming growth factor-beta (TGF-β), basic fibroblast growth factor (bFGF), vascular endothelial growth factor (VEGF), and endothelial growth factor (EGF). As a result of increased platelet and white blood cell counts in PRP, increased concentrations of growth factors were found.

**Table 2. Growth Factor Content of Blood and PRP**

| **Mean ± SD; n = 9** | **Initial Blood** | **PRP** |
|---|---|---|
| PDGF-AB (ng/mL) | 10.2 ± 1.4 | 88.4 ± 28.8 |
| PDGF-AA (ng/mL) | 2.7 ± 0.5 | 22.2 ± 4.2 |
| PDGF-BB (ng/mL) | 5.8 ± 1.4 | 57.8 ± 36.6 |
| TGF-β1 (ng/mL) | 41.8 ± 9.5 | 231.6 ± 49.1 |
| bFGF (pg/mL) | 10.7 ± 2.9 | 48.4 ± 25.0 |
| VEGF (pg/mL) | 83.1 ± 65.5 | 597.4 ± 431.4 |
| EGF (pg/mL) | 12.9 ± 6.2 | 163.3 ± 49.4 |

### Example 2

### Autologous Platelet Gel Generation

Autologous Platelet Gel (APG) is generated from the PRP fraction produced in the Magellan system by adding thrombin and calcium to activate the fibrinogen present in the PRP. For each approximately 7-8 mL of PRP, approximately 5000 units of thrombin in 5 mL 10% calcium chloride are required for activation. The APG is formed immediately upon mixing of the activator solution with the PRP. The concentration of thrombin can be varied from approximately 1-1,000 U/mL, depending on the speed required for setting to occur. The lower concentrations of thrombin will provide slower gelling times.

### Example 3

### Effects of APG on Cell Proliferation

A series of in vitro experiments were conducted evaluating the effect of released factors from APG on the proliferation of the human microvascular endothelial cells, human coronary artery smooth muscle cells and human dermal fibroblasts. Primary cell cultures of the three cell types were established according to protocols well known to those skilled in the art of cell culture. For each cell type, three culture conditions were evaluated. For APG cultures, APG was added to cells in basal medium. A second group of cells were cultured in growth medium. Growth medium is the standard culture medium for the cell type and contains optimal growth factors and supplements. The control cultures contain cells cultured only in basal medium which contains no growth factors.

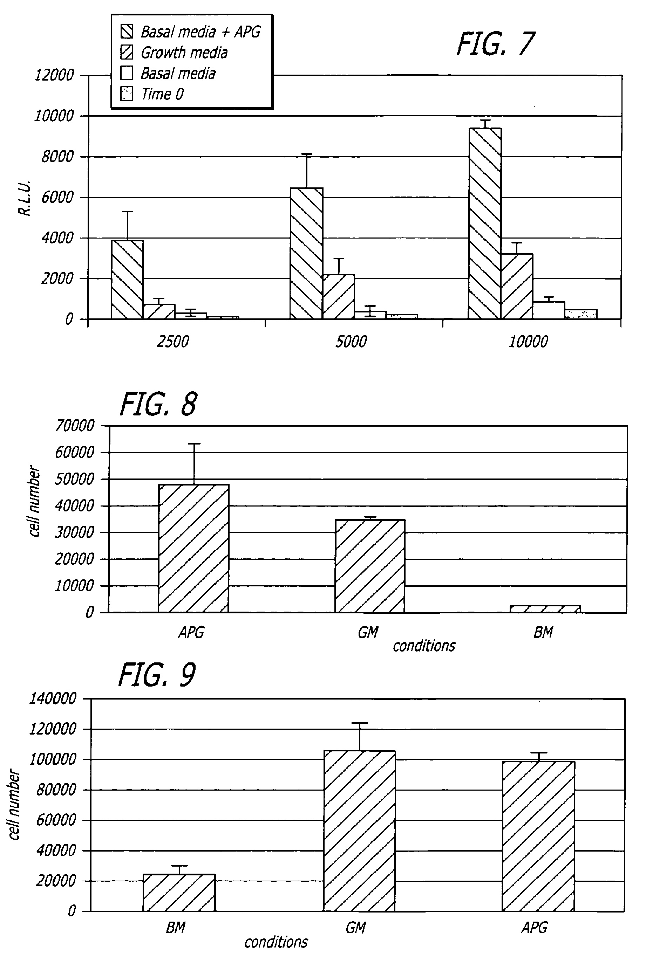

Autologous platelet gel had a significant growth effect on human coronary artery smooth muscle cells after five days of culture (FIG. 7), human microvascular endothelial cells after four days of culture (FIG. 8) and on human dermal fibroblasts after five days of culture (FIG 9).

### Example 4

### Effect of Platelet Poor Plasma on Human Dermal Fibroblast Growth

In addition to the platelet-rich plasma fraction, the Magellan system generates a platelet-poor plasma (PPP) fraction as well. This PPP fraction was further processed by centrifuging at 10,000xg for 10 minutes. The PPP fractions were then activated with the CaCl₂/thrombin activator solution used in the APG generation. Human dermal fibroblasts were then cultured in basal medium containing PRP gel or PPP gel. Culture condition for proliferation of human dermal fibroblasts are well known to those of ordinary skill in the art of cell culture.

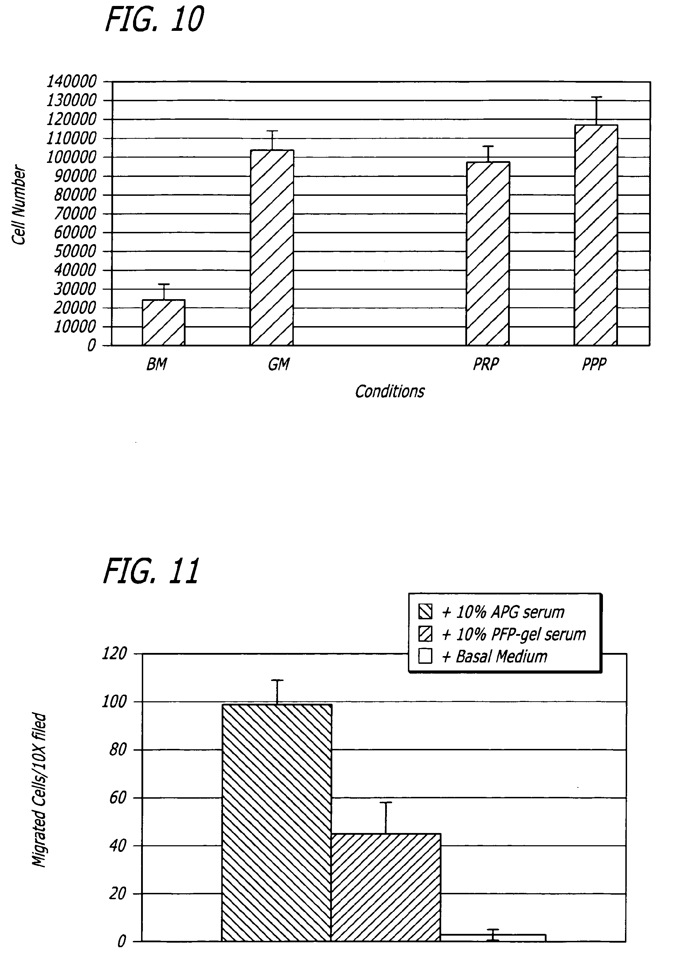

Human dermal fibroblasts cultured in the presence of PPP gel proliferated to a similar extent as those cultured in the presence of PRP gel (FIG. 10).

### Example 5

### Effect of APG on Endothelial Cell Migration

Human microvascular endothelial cell migration was performed in a Boyden chemotaxis chamber which allows cells to migrate through 8 µm pore size polycarbonate membranes in response to a chemotactic gradient. Human microvascular endothelial cells (5×10⁵) were trypsinized, washed and resuspended in serum-free medium (DMEM) and 400 µL of this suspension was added to the upper chamber of the chemotaxis assembly. The lower chamber was filled with 250 µL serum-free DMEM containing either 10% APG-derived serum, 10% platelet-free plasma (PFP)-derived serum or DMEM alone. After a pre-determined amount of time, the filters were removed and the cells remaining on the upper surface of the membrane (cells that had not migrated through the filter) were removed with a cotton swab. The membranes were then sequentially fixed, stained and rinsed to enable the visualization and quantification of cells that had migrated through the pores to the other side of the membrane. Autologous platelet gel-derived serum induced significantly more migration in human microvascular endothelial cells than either PFP or basal medium (FIG. 11).

### Example 5

### Effect of APG on Neovascularization in Athymic Mice

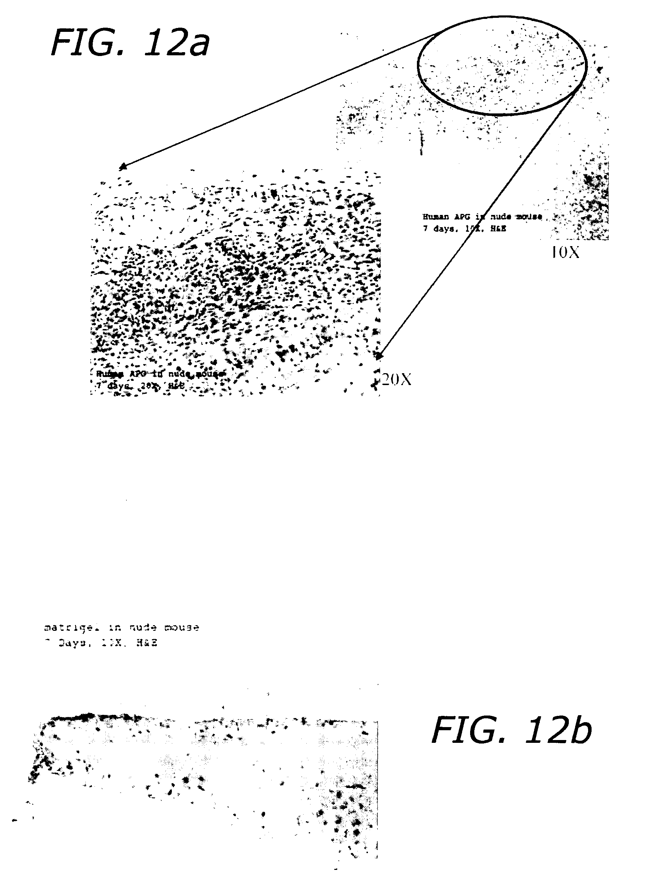

Autologous platelet gel was injected subcutaneously in nude (athymic) mice to determine if the APG is detectable and retrievable after a seven day implantation period. Athymic mice were injected with 500 µL of either APG or an inert Matrigel® control. Each animal was injected with Matrigel® in the left flank and APG in the right flank. After seven days the implants and the surrounding tissue were subjected to histological analysis (FIG. 12). In the area of the Matrigel® control implant there was minimal reaction to the material and a very thin capsule of loose connective tissue rimmed the mass (FIG. 12a). In the area of the APG implant, the APG was deeply infiltrated by spindle shaped cells (fibroblasts and macrophages) along with moderate numbers of neutrophils (FIG. 12b). The entire mass was rimmed by a thick layer of fibrovascular tissue and the tissue showed significant neovascularization.

According to an embodiment, a method for providing a stent graft and a cell growth promoting composition is provided, said method comprising: administering said cell growth promoting composition directly to a lumen of a blood vessel wall adjacent to a treatment site; and contacting said cell growth promoting composition with said luminal wall contacting surface of said stent graft and said blood vessel luminal wall at said treatment site. The cell growth promoting composition may be autologous platelet gel. Furthermore, a drug may be provided in combination with said autologous platelet gel. The drug may be selected from the group consisting of small molecules, peptides, proteins, hormones, DNA or RNA fragments, cells, genetically engineered cells, genes, cell growth promoting compositions and matrix metalloproteinase inhibitors. The method may further comprise advancing a stent deploying catheter containing a vascular stent to a treatment site; advancing at least one injection catheter containing said cell growth promoting composition to said treatment site; deploying said stent graft at said treatment site; and applying said cell growth promoting composition from said at least one injection catheter to said inner lumen of said blood vessel at said treatment site; such that said luminal wall contacting surface of said stent graft engages said cell growth promoting composition at said treatment site. The injection catheter may be selected from the group comprising single lumen injection catheter and multilumen injection catheter. Furthermore, a first of said at least one injection catheter may reach said treatment site through a different route than a second of said at least one injection catheter. Particularly, said first of said at least one injection catheter reaches said treatment site through a blood vessel bisecting the treatment site thereby delivering said cell growth promoting composition directly to the aneurysm sac. Typically, the treatment site is the area where the distal end of the stent graft contacts the vessel lumen wall or the junction between a stent graft and an iliac limb section or the aneurysm sac. According to an even further embodiment, a method for treating abdominal aortic aneurysms is provided, said method comprising delivering a stent graft to a treatment site; and promoting endothelialization of said stent graft by depositing a cell growth promoting composition on said inner lumen of said blood vessel contacting said luminal wall contacting surface of said stent graft and said inner lumen of said blood vessel; and promoting strengthening and fixation of stent graft by enhancing proliferation of smooth muscle cells and fibroblasts. Furthermore, the cell growth promoting composition may be autologous platelet gel.

Those skilled in the art will further appreciate that the embodiments according to the present invention may include other specific forms without departing from the spirit or central attributes thereof. In that the foregoing description discloses only exemplary embodiments, it is to be understood that other variations are contemplated as being within the scope of the present invention. Accordingly, the present invention is not limited in the particular embodiments which have been described in detail therein. Rather, reference should be made to the appended claims as indicative of the scope and content of the present invention.

## Claims

1. A stent graft comprising:
a luminal wall contacting surface;
a blood flow contacting surface; and
a cell growth promoting composition on said luminal wall contacting surface.

2. The stent graft of claim 1 wherein said cell growth promoting composition is applied to the exterior of said stent graft in a biocompatible polymer.

3. The stent graft of claim 1 or 2 wherein said cell growth promoting composition or said cell growth promoting composition containing-biocompatible polymer is applied directly to the exterior of said stent graft by spraying, dipping or rolling.

4. The stent graft of claim 1 wherein said cell growth promoting composition is applied directly to said luminal wall contacting surface of said stent graft in situ.

5. The stent graft of any of claims 1 to 4 wherein said cell growth promoting composition is autologous platelet gel (APG).

6. The stent graph of 5 wherein said APG or components of APG, such as platelet rich plasma (PRP), platelet poor plasma (PPP) or thrombin, are dispersed in a biocompatible polymer.

7. The stent graft of claim 5 or 6 wherein said stent graft is coated with APG prior to deployment by depositing PRP and thrombin on said stent graft in the stent graft chamber of a stent deployment catheter.

8. The stent graft of any of claims 1 to 4 wherein said cell growth promoting composition is an endothelial cell promoting factor, a smooth muscle cell promoting factor, or a fibroblast promoting factor.

9. The stent graft of claim 8 wherein said endothelial cell promoting factor consists of a vascular growth factor or a heparin binding growth factor.

10. The stent graft of claim 9 wherein said endothelial cell promoting factor is a vascular growth factor.

11. The stent graft of claim 9 wherein said endothelial cell promoting factor is a heparin binding growth factor.

12. The stent graft of any one of claims 9 or 10 wherein said vascular growth factor is selected from the group consisting of vascular endothelial growth factor A, vascular endothelial growth factor B, vascular endothelial growth factor C, vascular endothelial growth factor D and placental growth factor.

13. The stent graft of any one of claims 9 or 11 wherein said heparin binding growth factor is selected from the group consisting of fibroblast growth factor 1, fibroblast growth factor 2 and insulin-like growth factor.

14. The stent graft of claim 1 wherein said stent graft further comprises a metal scaffolding attached to said luminal wall contacting surface.

15. A delivery catheter for delivering a stent graft and a cell growth promoting composition to an aneurysm site comprising:
a stent graft chamber in which said stent graft is radially compressed;
an injection catheter disposed within said delivery catheter; and
at least one injection port at the distal end of said injection catheter capable of delivering said cell growth promoting composition.

16. The delivery catheter of claim 15 wherein said injection catheter is selected from the group comprising single lumen injection catheter and multilumen injection catheter.

17. The delivery catheter according to claim 15 or 16 wherein said injection catheter further comprises a vent for expressing air or excess cell growth promoting composition.
